# EUROPÄISCHE PATENTANMELDUNG

(11) **EP 2 033 619 A1**
(43) Veröffentlichungstag der Anmeldung: **11.03.2009**
(21) Anmeldenummer: 07114920.7
(22) Anmeldetag: 24.08.2007
(51) Int. Cl.: A61K 8/11, A61K 8/19, A61K 8/27, A61K 8/29, A61K 8/64, A61K 8/98, A61K 9/16, A61K 9/20, B01F 17/00, B01J 13/22, C09C 3/10, C09D 5/00, C09J 7/00, A61Q 17/04

(54) **Verfahren zum Dispergieren von ionischen Nanopartikeln**

(71) Anmelder: BASF SE, 67056 Ludwigshafen (DE)
(72) Erfinder: Wohlleben, Wendel, Dr., 68165 Mannheim (DE)
(74) Vertreter: Huhn, Michael

(57) **Zusammenfassung**

Verfahren zum Dispergieren von ionischen Nanopartikeln in einem wässrigen Medium, wobei man ionische Nanopartikel in Gegenwart eines Dispergiermittels aus der Gruppe der globulären Proteine in einem wässrigen Medium dispergiert, eignet sich zum Herstellen von Dispersionen zur Verwendung in Kosmetika, Arzneimitteln, Lacken, Klebern und thermoplastischen Formmassen.

## Beschreibung

Die Erfindung betrifft ein Verfahren zum Dispergieren von ionischen Nanopartikeln in wässrigen Medien, nach einem solchen Verfahren erhältliche Dispersionen sowie deren Verwendung in kosmetischen und pharmazeutischen Anwendungen sowie Kunststoffen.

Ionische Nanopartikel, d.h. Partikel mit einem Durchmesser von 1 bis 1000 nm, haben in jüngerer Zeit Einsätze in kosmetischen Cremes, wie Sonnencremes, Pasten, Lacken, Klebern, aber auch Nahrungsmitteln ("Nano-Food") gefunden. Darüber hinaus wird auch die Verwendung in pharmazeutischen Zubereitungen sowie thermoplastischen Formmassen vorgeschlagen. Dazu werden die Nanopartikel häufig in Form wässriger Dispersionen eingesetzt.

Um ionische Nanopartikel in wässrige Medien einbringen zu können, sind hohe mechanische Kräfte erforderlich. Es ist üblich, Dispergiermittel einzusetzen, um diese Dispergierkräfte zu erniedrigen und um den zur Deflockulierung der Feststoffteilchen notwendigen Gesamtenergieeintrag in das System und damit auch die Dispergierzeit so gering wie möglich zu halten. Hierbei handelt es sich meist um oberflächenaktive Stoffe von anionischer, kationischer oder neutraler Struktur. Die Stoffe werden in kleiner Menge entweder direkt auf den Feststoff aufgebracht oder dem Dispergiermedium zugesetzt. Bekannt ist weiterhin, dass es auch nach vollständiger Deflockulierung der Feststoffagglomerate in Primärteilchen nach dem Dispergierprozess zu Reagglomerationen kommt, wodurch der Dispergieraufwand teilweise oder vollständig zunichte gemacht wird. Als Folge der unzulänglichen Dispergierung oder durch Reagglomeration kommt des zu unerwünschten Effekten wie Viskositätsanstieg in flüssigen Systemen, Farbtondrift und Glanzverlusten in Lacken und Beschichtungen sowie Verringerung der mechanischen Festigkeit in Kunststoffen.

Eine Vielzahl verschiedener Substanzen findet heute Verwendung als Dispergiermittel beispielsweise für feinverteilte Pigmente und Füllstoffe. Neben niedermolekularen Verbindungen wie Lecithin, Fettsäuren und deren Salzen und Alkylphenolethoxylaten werden auch komplexe Strukturen als Dispergiermittel eingesetzt.

Die bekannten Systeme zeigen jedoch Nachteile, beispielsweise dass sie jeweils nur für ein eng begrenztes Einsatzgebiet verwendet werden können, Schaumbildner sind oder, wie die Alkylphenolethoxylate, ökobiologische Risiken aufgrund ihrer speziellen chemischen und physikalischen Eigenschaften bergen.

Es besteht daher die ständige Aufgabe, neue Dispergiermittel zu entwickeln, welche die Nachteile der bekannten Systeme vermeiden oder zumindest vermindern.

Es ist beschrieben (D. Nepal und K. E. Gecheler, small 3 (2007) 1255-126f, H Hyung et al., Environ. Sci. Technol., 41 (1), S. 179 - 184,), dass unpolare Kohlenstoffnanoröhren durch bestimmte Proteine und natürliche organische Materie in wässriger Phase stabilisiert werden. Eine Eignung solcher Materialien zum Dispergieren hochpolarer ionischer Nanopartikeln lässt sich daraus nicht ableiten.

In der WO 2007/006765 sind wässrige Momomeremulsionen enthaltend anorganische Teilchen als Pickering-Emulgatoren und Hydrophobin-Proteine beschrieben. Eine spezifische Eignung der Hydrophobine zum Dispergieren von anorganischen Nanopartikeln geht aus dem Dokument nicht hervor.

Es wurde nun gefunden, dass globuläre Proteine in besonderer Weise zum Dispergieren von ionischen Nanopartikeln in wässrigen Systemen geeignet sind.

Gegenstand der Erfindung ist daher ein Verfahren zum Dispergieren von ionischen Nanopartikeln in einem wässrigen Medium, wobei man ionische Nanopartikel in Gegenwart eines Dispergiermittels aus der Gruppe der globulären Proteine in einem wässrigen Medium suspendiert.

Weiterhin Gegenstand der Erfindung sind wässrige Suspensionen ionischer Nanopartikel erhältlich nach dem erfindungsgemäßen Verfahren, sowie deren Verwendung bei der Herstellung von kosmetischen und pharmazeutischen Zusammensetzungen und Kunststoffen.

Ebenso Gegenstand der Erfindung ist die Verwendung von globulären Proteinen als Dispergiermittel für ionische Nanopartikeln in einem wässrigen Medium.

Durch Einsatz der erfindungsgemäßen Dispergiermittel lassen sich eine Vielzahl ionischer Nanopartikel durch einfaches Rühren ohne zusätzlichen Energieeintrag in wässrigen Medien dispergieren, ohne dass es zu unerwünschten Agglomerationen kommt. Zudem sind die verwendeten Dispergiermittel ungiftig und biologisch abbaubar.

Dispergieren im Sinne der Erfindung bedeutet, dass es zwischen dem Dispergiermittel und den Nanopartikeln lediglich zu physikalischen Wechselwirkungen, beispielsweise zu einer Adsorption des Dispergiermittels auf der Oberfläche der Nanopartikel, nicht aber zur Ausbildung einer chemischen Bindung kommt. Als ionische Nanopartikel im Sinne der Erfindung gelten feinteilige Materialien, die aus mindestens einem Metall und/oder Halbmetall und mindestens einem Nichtmetall aufgebaut sind, mit einem durchschnittlichen zahlenmittleren Primärteilchendurchmesser d₅₀ (je 50 % der Teilchen sind kleiner oder größer als der d₅₀-Durchmesser) von 0,1 bis 1000 nm, bevorzugt 0,5 bis 250 nm, besonders bevorzugt 1 bis 100 nm, insbesondere 5 bis 40 nm (bestimmt durch Transmissionselektronenmikroskopie (TEM) und Auswertung nach bildanalytischen Methoden).

Als ionische Nanopartikel eignen sich im Allgemeinen Teilchen aus der Gruppe der Oxide, Sulfide, Selenide, Telluride, Halogenide, Carbide, Arsenide, Antimonide, Nitride, Phosphide, Carbonate, Carboxylate, Phosphate, Sulfate, Silikate, Titanate, Zirkonate, Aluminate, Stannate, Plumbate sowie Mischoxide davon.

Ionische Nanopartikel als solche sind bekannt, z.B. aus der WO 96/31571, und sind beispielsweise Oxide wie CaO, ZnO, CdO, SiO₂, TiO₂, ZrO₂, CeO₂, SnO₂, PbO, Al₂O₃, In₂O₃ und La₂O₃; Sulfide wie CdS und ZnS; Selenide wie GaSe, CdSe oder ZnSe; Telluride wie ZnTe oder CdTe; Halogenide wie NaCl, KCI, BaCl₂, AgCl, AgBr, Agl, CuCl, CUBr, CdI₂ oder PbI₂; Carbide wie CeC₂; Arsenide wie AlAs, GaAs oder CeAs; Antimonide wie InSb; Nitride wie BN, AIN, Si₃N₄ oder Ti₃N₄; Phosphide wie GaP, InP, Zn₃P₂ oder Cd₃P₂; Carbonate wie Na₂CO₃, K₂CO₃, CaCO₃, SrCO₃ und BaCO₃, Carboxylate, z.B. Acetate wie CH₃COONa und Pb(CHCOO)₄; Phosphate; Sulfate; Silicate; Titanate; Zirkonate; Aluminate; Stannate; Plumbate und entsprechende Mischoxide, deren Zusammensetzung vorzugsweise der Zusammensetzung herkömmlicher Gläser mit niedrigem thermischen Ausdehnungskoeffizienten entspricht, z.B. binäre, tertiäre oder quaternäre Kombinationen von SiO₂, TiO₂, ZrO₂ und Al₂O₃. Ebenfalls geeignet sind z.B. Mischoxide mit Perowskit-Struktur wie BaTiO₃ oder PbTiO₃. Außerdem können organisch modifizierte anorganische Teilchen wie partikuläre Polymethylsiloxane, methacrylfunktionalisierte Oxidpartikel und Salze der Methylphosphorsäure verwendet werden.

Die ionischen Nanopartikel umfassen vorzugsweise Oxide, insbesondere ZnO, CdO, SiO₂, TiO₂, ZrO₂, CeO₂, SnO₂, Al₂O₃, In₂O₃, La₂O₃, Fe₂O₃, Ta₂O₅, Cu₂O, V₂O₅, MoO₃ und WO₃.

Besonders bevorzugt sind ZnO, TiO₂, ZrO₂ und CeO₂, insbesondere ZnO und TiO₂.

Die Herstellung dieser ionischen Nanopartikel kann auf übliche Weise erflogen, z.B. durch Sol-Gel-Verfahren, Flammhydrolyse, Flammpyrolyse und Plasmaverfahren gemäß der in der WO 96/31 572 genannten Literatur. Besonders bevorzugt sind kolloidale nanodisperse Sole und Pulver von anorganischen Teilchen wie z.B. Kieselsole der Fa. H.C. Starck, SnO₂-Sole der Fa. Goldschmidt, TiO₂-Sole der Fa. MERCK, SiO₂-, ZrO₂-, Al₂O₃-, Sb₂O₃-Sole der Fa. Nissan Chemicals oder Aerosildispersionen der Fa. DEGUSSA, oder CeO₂ und ZnO-Sole der BASF Aktiengesellschaft.

Die ionischen Nanopartikel können durch Oberfllächenmodifikation hinsichtlich verschiedener Eigenschaften, wie Viskosität, Oberflächenenergie, Hydrophilie verändert werden.

Beispielsweise können die ionischen Nanopartikel einer Oberflächenmodifikation mit siliciumorganischen Verbindungen unterzogen werden. Die an der Oberfläche modifizierten Teilchen sind in ihrer Reaktivität mit und Affinität für das Matrixharz und auch in ihrer Affinität für Lösungsmittel oder Dispersionsmedien verbessert. Die siliciumorganischen Verbindungen, die für den obigen Zweck verwendet werden, schließen zum Beispiel monofunktionale Silane der Formel R₃SiX ein, worin jedes R eine organische Gruppe mit einer Alkylgruppe, einer Phenylgruppe, einer Vinylgruppe, einer Methacry-Ioxygruppe, einer Mercaptogruppe, einer Aminogruppe oder einer Epoxygruppe darstellt und X eine hydrolysierbare Gruppe darstellt. Spezifische Beispiele schließen Trimethylmethoxysilan, Phenyldimethylmethoxysilan, Phenyldimethylethoxysilan, Vinyldimethylmethoxysilan, Vinyldimethylethoxysilan, γ-Acryloxypropyldimethylmethoxysilan, γ-Methacryloxypropyldimethylmethoxysilan, γ-Mercaptopropylmethyldiethoxysilan, N-γ-(Aminoethyl)-γ-aminopropyldimethyldimethoxysilan, γ-Aminopropyldimethylmethoxysilan, γ-Aminopropyldimethylethoxysilan, γ-Glycidoxypropyldimethylmethoxysilan, γ-Glycidoxypropyldemthoxyethoxysilan, β-(3,4-Epoxycyclohexyl)ethyldimethylmethoxysilan und dergleichen ein. Alternativ können auch bifunktionale Silane der Formel R₂SiX₂ verwendet werden, worin jedes R und jedes X dieselben Bedeutungen besitzt, wie oben definiert. Spezifische Beispiele schließen Dimethydimethoxysilan, Diethyldimethoxysilan, Dimethyldiethoxysilan, Diethyldiethoxysilan, Diphenyldimethoxysilan, Phenylmethyldimethoxysilan, Phenylmethyldiethoxysilan, Vinylmethyldimethoxysilan, Vinylmethyldiethoxysilan, γ-Mercaptopropylmethyldimethoxysilan, γ-Mercaptopropylmethyldiethoxysilan, N-β-(Aminoethyl)-γ-aminopropylmethyldimethoxysilan, γ-Aminopropylmethyldimethoxysilan, γ-Aminopropylmethyldiethoxysilan, γ-Glycidoxypropylmethylmethoxysilan, γ-Glycidoxypropylmethyldiethoxysilan, β-(3,4-Epoxycyclohexyl)ethylmethyldimethoxysilan und dergleichen ein. In einer weiteren Alternative können trifunktionale Silane der Formel RSiX₃ verwendet werden, worin jedes R und jedes X dieselben Bedeutungen hat, wie oben definiert. Spezifische Beispiele schließen Methyltrimethoxysilan, Ethyltrimethoxysilan, Methyltriethoxysilan, Ethyltriethoxysilan, Phenyltrimethoxysilan, Phenyltriethoxysilan, Vinyltrimethoxysilan, Vinyltriethoxysilan, Vinyl(β-methoxyethoxy)silan, γ-Acryloxypropyltrimethoxysilan, γ-Methacryloxypropyltrimethoxysilan, γ-Mercaptopropyltrimethoxysilan, γ-Mercaptopropyltriethoxysilan, N-β-Aminoethyl)-γ-aminopropyltrimethoxysilan, γ-Aminopropyltrimethoxysilan, γ-Aminopropyltriethoxysilan, γ-Glycidoxypropyltrimethoxysilan, β-Glycidoxypropyltrimethoxysilan, γ-Glycidoxypropyltriethoxysilan, β-Glycidoxypropyltriethoxysilan, β-(3,4-Epoxycyclohexyl)ethyltrimethoxysilan und dergleichen ein.
In einer weiteren Alternative können trifunktionale Silane der Formel SiX₄ verwendet werden, worin X dieselbe Bedeutung hat, wie oben definiert. Spezifische Beispiele schließen Tetraethylorthosilicat, Tetramethylorthosilicat und dergleichen ein.

Erfindungsgemäß wird zum Benetzen und Dispergieren ein Dispergiermittel aus der Gruppe der globulären Proteine und Phospholipide eingesetzt.

Als Protein eignen sich im Allgemeinen globuläre Proteine (Sphäroproteine) mit einem Molgewicht von ungefähr 500 bis 100.000. Der isoelektrische Punkt liegt im Allgemeinen bei 4,00 bis 12,00.

Bevorzugt sind weiterhin Proteine mit verschiedenen Bindungsstellen für Oberflächen unterschiedlicher chemischer Struktur, wie polaren und unpolaren, hydrophilen und hydrophoben Bindungsstellen.

Bevorzugte Klassen von Proteinen umfassen:
a) Serumproteine, bevorzugt Fetuine, Albumine, besonders bevorzugt Rinderserumalbumine (BSA, bovine serum albumin), verdünntes fötales Rinderserum (FCS), Humanerserum Albumin (HSA) und Ovalbumin (OvB);
b) Histon (HAST) und Histon-ähnliche Proteine;
c) Hämoglobin (HBA);
d) Myoglobin (MGB);
e) Trypsin (TPS);
f) Glucoseoxidase (GOD);
g) Antikörper und Immunoglobuline, bevorzugt Immunoglobulin G (IgG);
h) Hydrophobine, bevorzugt H*Protein A, H*Protein B und SC3;
i) Lungen Surfactant Proteine, beispielsweise erhalten durch Lungenlavase (Lungenspülung), bevorzugt Surfuctant Proteine SPA, SP-B und SP-C, unmodifizierte oder in derivatisierter Form, beispielsweise verestert, oder deren synthetische Ersatzstoffe wie Lusupultide, insbesondere rekombinant hergestellt, wobei die genannten Proteine vorzugsweise als Suspensionen, sprühgetrocknet oder in Pulverform eingesetzt werden.

Die genannten Proteine sind bekannt und beispielsweise beschrieben in J. M. Berg, J. L. Tymoezko, L. Stryer, Biochemistry, 6. Aufl., Palgrave Macmillan 2006.

Sie sind teilweise kommerziell erhältlich, beispielsweise Histon, Hämoglobin, Myoglobin, Ovalbumin, Rinderserumalbumin, Trypsin und Glucoseoxidase von der Sigma Aldrich GmbH, München, Deutschland. Die Herstellung von Hydrophobinen ist beispielsweise in der WO 2007/006765, WO 2006/103215 und der dort zitierten Literatur beschrieben.

Es können natürlich auch Mischungen verschiedener globulärer Proteine eingesetzt werden.

Erfindungsgemäß werden Proteine als Dispergiermittel zur Herstellung wässriger Suspensionen von ionischen Nanopartikeln eingesetzt. Die Zugabe des Dispergiermittels kann dabei vor, während oder nach der Zugabe der Nanopartikel erfolgen, bevorzugt ist die Zugabe des Dispergiermittels vor Zugabe der Nanopartikel.

Das Gewichtsverhältnis von Dispergiermittel zu Nanopartikeln kann in breitem Rahmen variieren. Bevorzugt beträgt das Verhältnis von Dispergiermittel zu Nanopartikeln 30:1 bis 1:20, besonders bevorzugt 3:1 bis 1:20, insbesondere 1:1 bis 1:10.

Das Dispergieren der Nanopartikel erfolgt im Allgemeinen durch Rühren, es kann aber beispielsweise auch durch Behandlung mit Ultraschall erfolgen. Die Reaktionsdauer beträgt im Allgemeinen 0,01 bis 24 h, vorzugsweise 0,1 bis 2 h.

Das Dispergieren findet im Allgemeinen bei Raumtemperatur statt, kann aber auch bei erhöhter Temperatur, stattfinden, solange kein Denaturieren der Proteine eintritt.

Gegenstand der Erfindung sind auch die nach dem erfindungsgemäßen Verfahren erhältlichen wässrigen Suspensionen.

Bevorzugte weitere Bestandteile sind beispielsweise Verdicker, Säuren und Basen zur Einstellung eines bestimmten pH-Wertes, der z.B. für die Dispergiermischung eines bestimmten Proteins günstig ist.

Die erfindungsgemäßen Suspensionen eignen sich beispielsweise zur Einarbeitung in Formulierungen und Emulsionen, insbesondere für kosmetische-, medizinische- und Nahrungsmittelanwendungen.

Weiterhin können diese Suspensionen zur Herstellung von Kunststoffen verwendet werden, wobei beispielsweise Pigmente oder Füllstoffe in feinstverteilter Form eingebracht werden. Bevorzugt ist die Verwendung zur Herstellung wässriger Kunsstoffdispersionen, beispielsweise bei der Herstellung von wasserbasierenden Lachen und Klebern.

Bevorzugt ist auch die Verwendung zur Herstellung von thermoplastischen Formmassen, wobei die erfindungsgemäßen Suspensionen bei der Sythese der thermoplastischen Polymere durch Dispersionspolymerisation zugesetzt werden.

Geeignete Komponenten und Additive sind beispielsweise in der EP-A 1 783 170 beschrieben, deren Inhalt durch Zitat als Bestandteil dieser Beschreibung gilt.

Neben dem erfindungsgemäßen Einsatz von Proteinen und Phospholipiden als Dispergierungmittel und als Dispersionsstabilisatoren ist auch die Beschichtung von pulver- oder faserförmigen Nanopartikeln mit den erfindungsgemäßen Dispergiermitteln Gegenstand der Erfindung. Derartige Beschichtungen werden in bekannter Weise ausgeführt, wie z.B. in EP-A-0 270 126 beschrieben. Hierbei kann das Lösungs- oder Emulsionsmittel entweder entfernt werden oder im Gemisch unter Bildung von Pasten verbleiben. Diese Pasten sind übliche Handelsprodukte und können zusätzlich Bindemittelanteile sowie weitere Hilfs- und Zusatzstoffe enthalten. Speziell bei Pigmenten kann die Beschichtung der Pigmentoberfläche während oder nach der Synthese der Pigmente erfolgen, z.B. durch Zusatz der erfindungsgemäßen Dispergiermittel zur Pigmentsuspension oder während oder nach dem Pigmentfinish.

Die auf diese Weise vorbehandelten Nanopartikel, insbesondere Pigmente, zeichnen sich durch leichtere Einarbeitbarkeit im Bindemittel, sowie durch verbessertes Viskositäts-, Flockulations- und Glanzverhalten gegenüber nicht behandelten Nanopartikeln aus.

Gegenstand der Erfindung sind daher auch ionische Nanopartikel, beschichtet mit einem globulären Protein.
Die Erfindung wird durch die Beispiele näher erläutert ohne sie dadurch einzuschränken.

### Beispiele

Jeweils 0,5 mg verschiedener nanopartikulärer Materialien wurden mit 5 ml Wasser versetzt und mit oder ohne Zugabe verschiedener Dispergiermittel 24 h gerührt. Benetzung und Aggregationsgrad der dispergierten Teilchen wurden bestimmt.

Die Teilchengrößenverteilung wurde durch analytische Ultrazentrifugation mit Trübungsdetektor (H. Cölfen, "Analytical Ultracentrifugation of Nanoparticles", in Encyclopedia of Nanoscience and Nanotechnology, (American Scientific Publishers, 2004), S. 67-88.) oder Interferenzdetektor (W. Mächtle and L. Börger, "Analytical Ultracentrifugation of Polymers and Nanoparticles, (Springer, Berlin, 2006). ) bestimmt.

Zur Auswertung der Rohdaten wurde die Stokes-Einstein-Beziehung verwendet, wobei sich
D=SQRT(18µs/ Δp) ,
der kugeläquivalente Durchmesser D ergibt. Dabei ist µ die bekannte Viskosität des Dispergiermediums, s der gemessene Sedimentationskoeffizient und Δρ die Dichtedifferenz zwischen dem Nanopartikel und dem Dispergiermedium.(vgl. H. Cölfen, "Analytical Ultracentrifugation of Nanoparticles", in Encyclopedia of Nanoscience and Nanotechnology, (American Scientific Publishers, 2004), S. 67-88.) In der folgenden Tabelle werden die Durchmesser D10 und D50 angegeben. Diese sind dadurch definiert, dass 10 Gew.-% der Partikel (ohne Berücksichtigung der Dispergiermittel) einen kleineren Durchmesser als D10 haben, entsprechend 50 Gew.-% kleiner als D50. D50 ist damit der gewichtsmittlere Durchmesser.

Folgende Materialien wurden eingesetzt:
- TiO₂ P25 (Hersteller DEGUSSA, zahlenmittlerer Primärpartikeldurchmesser 21 nm nach TEM Messung)
- CeO₂ (Hersteller BASF, zahlenmittlerer Primärpartikeldurchmesser unter 50 nm nach TEM-Messung)
- ZnO (Hersteller BASF, zahlenmittlerer Primärpartikeldurchmesser unter 50 nm nach TEM-Messung)
- ZrO₂ (modifiziert mit Polyoxalsäure, Hersteller ITN, zahlenmittlerer Primärpartikeldurchmesser 15 nm nach TEM)
- Mersolat®-K30 (Natriumalkansulfonat, Dispergiermittel, Hersteller Lanxess)
- Rinderserumalbumin (BSA) (Hersteller Sigma-Aldrich)
- Fötales Rinderserum (FCS) (Hersteller Sigma-Aldrich)
- H*Protein A (Hydrophobin, erhalten gemäß WO 2007/006765)

**Tabelle 1**

| Dispergieren von ionischen Nanopartikeln mit Proteinen | | | | | | |
|---|---|---|---|---|---|---|
| Beispiel Nr | Nano-partikel | Dispergiermittel | Verhältnis Nanopartikel/Dispersion smittel | D10 / nm | D50 / nm | Bemerkung |
| V1 | TiO2 | - | | | | keine Benetzung, keine Dispergierung |
| V2 | CeO2 | - | | | | keine Benetzung, keine Dispergierung |
| V3 | ZnO | - | | | | keine Benetzung, keine Dispergierung |
| V4 | TiO2 | Mersolat®-K30 | 01:50 | | > 1000 | Benetzung, überw. Agglomerate |
| V5 | CeO2 | Mersolat®-K30 | 01:50 | | > 1000 | Benetzung, überw. Agglomerate |
| V6 | ZnO | Mersolat®-K30 | 01:50 | | > 1000 | Benetzung, überw. Agglomerate |
| 1 | TiO2 | BSA | 01:30 | 46 | 99 | benetzt |
| 2 | TiO2 | BSA | 01:05 | 54 | 210 | benetzt |
| 3 | TiO2 | BSA | 01:01 | 99 | 220 | benetzt |
| 4 | TiO2 | BSA | 10:01 | 88 | 330 | benetzt |
| 5 | TiO2 | BSA | 20:01 | 180 | 380 | benetzt |
| 6 | TiO2 | FCS | | | 32 | benetzt |
| 7 | CeO2 | FCS | | | 27 | benetzt |
| 8 | ZnO | FCS | | | 30 | benetzt |
| 9 | TiO2 | H*Protein A | 01:01 | 60 | 180 | benetzt |

| | | | | | | |
|---|---|---|---|---|---|---|
| (V=Vergleichsversuch) | | | | | | |

Die Versuche belegen eine gute Dispergierwirkung der erfindungsgemäßen Dispergiermittel. Als Referenzversuch dient zunächst die Dispergierung in reinem Wasser (V1-3): Hier wird ohne Ultraschall überhaupt keine Dispergierung erreicht, so dass auch keine Aggregatgröße gemessen werden kann. Als nächste Referenz dient die Dispergierung mit dem nicht-biologischen Standard-Dispergiermittel Mersolat®-K30 (V4-6). Hier wird eine Benetzung erreicht, jedoch liegen die Partikel in großen Aggregaten mit einem mittleren Durchmesser von über 1µm vor. Dagegen wird in den Mischungen mit BSA und H*Protein A (Beispiele 1-4 und 9) bereits durch einfaches Rühren (ohne Ultraschall) eine gute Dispergierung erreicht, wobei mindestens 10 % der Partikel eine mittlere Größe von unter 100 nm aufweisen. Dies bedeutet eine deutliche Verbesserung, da bei den Beispielen V1-V6 überhaupt keine Partikel im Bereich unter 100 nm entstehen. Die Beispiele 6-9 belegen, dass in reinem FCS sogar eine Dispergierung bis auf isolierte Primärpartikel erreicht wird, denn 50% der Partikel erreichen Durchmesser von unter 30 nm.

## Patentansprüche

1. Verfahren zum Dispergieren von ionischen Nanopartikeln in einem wässrigen Medium, wobei man ionische Nanopartikel in Gegenwart eines Dispergiermittels aus der Gruppe der globulären Proteine in einem wässrigen Medium dispergiert.

2. Verfahren gemäß Anspruch 1, wobei man oxidische Nanopartikel einsetzt.

3. Verfahren gemäß Anspruch 2, wobei die oxidischen Nanopartikel aus der Gruppe ZnO, CdO, SiO₂, TiO₂, ZnO₂, CeO₂, SnO₂, Al₂O₃, In₂O₃, La₂O₃, Fe₂O₃, Ta₂O₅, Cu₂O, V₂O₅, MnO₃ und WO₃ gewählt sind.

4. Verfahren gemäß einem der Ansprüche 1 bis 3, wobei das globuläre Protein aus der Gruppe der Serumproteine (einschließlich verdünnter Seren), Histone, Hämoglobin, Myoglobin, Trypsin, Glucoseoxidase, Antikörper und Immunglobuline, Hydrophobine und Lungen-Surfactant-Proteine gewählt wird.

5. Verfahren gemäß Anspruch 4, wobei man als Dispergiermittel ein globuläres Protein aus der Gruppe der Albumine und Hydrophobine einsetzt.

6. Verfahren gemäß einem der Ansprüche 1 bis 5, wobei das Gewichtsverhältnis von Nanopartikeln zu Dispergiermittel 1:30 bis 20:1 beträgt.

7. Verwendung eines globulären Proteins zum Dispergieren von ionischen Nanopartikeln in einem wässrigen Medium.

8. Wässrige Suspension, erhältlich nach einem Verfahren gemäß einem der Ansprüche 1 bis 6.

9. Verwendung einer wässrigen Suspension gemäß Anspruch 8 zur Herstellung von kosmetischen Zubereitungen, pharmazeutischen Zubereitungen, Lacken, Klebern oder thermoplastischen Formmassen.

10. Ionische Nanopartikel, beschichtet mit einem Dispergiermittel aus der Gruppe der globulären Proteine.
